# EUROPEAN PATENT SPECIFICATION

(11) **EP 2 162 189 B1**
(45) Date of publication and mention of the grant of the patent: **07.03.2012**
(21) Application number: 08771042.2
(22) Date of filing: 13.06.2008
(51) Int. Cl.: A61P 15/10, A61K 35/12, A61M 1/00

(54) **METHODS OF RESTORATION OF ERECTILE FUNCTION**
VERFAHREN ZUR WIEDERHERSTELLUNG DER EREKTILEN FUNKTION
PROCÉDÉS DE RESTAURATION DE LA FONCTION ÉRECTILE

(30) Priority: 13.06.2007 US 943762 P
(43) Date of publication of application: 17.03.2010
(73) Proprietor: Wake Forest University Health Sciences, Winston-Salem NC 27101 (US)
(72) Inventor: YOO, James, Winston-Salem, NC 27104 (US); ATALA, Anthony, Winston-Salem, NC 27104 (US)
(74) Representative: Adam, Holger
(86) International application number: PCT/US2008/066944
(87) International publication number: WO 2008/157368

(56) References cited:
- WO-A2-2007/149548
- US-A1- 2003 050 228
- US-A1- 2003 119 714
- US-B1- 6 569 428
- SEFTEL ET AL: "Circulating Endothelial Progenitor Cells in Subjects With Erectile Dysfunction", JOURNAL OF UROLOGY, LIPPINCOTT WILLIAMS & WILKINS, BALTIMORE, MD, US, vol. 174, no. 2, 1 August 2005 (2005-08-01), page 656, XP005378791, ISSN: 0022-5347, DOI: DOI:10.1016/S0022-5347(01)68348-4
- GONZALEZ-CADAVID NESTOR F ET AL: "Therapy of erectile dysfunction: potential future treatments.", March 2004 (2004-03), ENDOCRINE 2004 MAR-APR LNKD- PUBMED:15146097, VOL. 23, NR. 2-3, PAGE(S) 167 - 176, XP002610325, ISSN: 1355-008X * page 172, column 2, paragraph first full *
- RODRIGUEZ S ET AL: "Progenitor-derived endothelial cell therapy restores erectile function", July 2007 (2007-07), TISSUE ENGINEERING, VOL. 13, NR. 7, PAGE(S) 1747-1748, CONFERENCE OF THE TISSUE-ENGINEERING-AND-REGENERATIVE-MEDICI NE-INTERN ATIONAL-SOCIETY (TERMIS-EU); LONDON, UK; SEPTEMBER 04 -07, 2007, XP009141401, ISSN: 1076-3279 * the whole document *
- BIVALACQUA T.J. ET AL.: 'Mesenchymal Stem Cells Alone OR ex vivo Gene Modification with Endothelial Nitric Oxide Synthase Reverse Age-Associated Erectile Dysfunction' AM. J. PHYSIOL. HEART CIRC. PHYSIOL. vol. 292, no. 3, October 2006, pages 1278 - 1290, XP008125128

## Description

### Background of the Invention

More than 600,000 middle-aged American men experience some degree of erectile dysfunction (ED) or impotence each year, resulting in approximately 20 to 30 million American men having ED at some point during their lives. Doctors estimate that up to 85% of ED cases are caused by medical or physical problems such as diabetes, high blood pressure, vascular disease, surgery to the prostate and urogenital area, spinal cord injury, multiple sclerosis, endocrine disorders, or medications. For example, approximately 35-75 % of men with diabetes mellitus are afflicted with ED, which accounts for approximately 30% of ED cases in the United States.

Currently used methods for the treatment of ED have included external devices, sex therapy, surgical implantation of internal prostheses, injection of drugs directly into the penis, oral medication (e.g., sildenafil, phenotalamine, alprostadil) and topically applied medications. None of these approaches is entirely effective and have a variety of side effects including headaches, diarrhea, flushing, hypotension, disturbed color vision, pain, or embarrassment. In addition, the use of such treatments induce temporary erections at the same time of administration, and thus only provide temporary relief of ED. Furthermore, the use of currently available oral medications for ED can be dangerous in men with cardiovascular disease since the drugs can affect blood pressure. According to the American Heart Association, ED in men with type 2 diabetes may be an early warning sign of heart disease. Therefore, many men afflicted with ED may also have heart disease and not realize the danger of taking oral medications.

Accordingly, a need exists for improved methods for ameliorating ED. In particular, a need exists for long term treatment of ED with minimal side effects.

### Summary of the Invention

The present invention provides methods and compositions for ameliorating erectile dysfunction using endothelial progenitor cells. In particular, methods are disclosed for maintaining intracavernosal pressure and ameliorating erectile dysfunction induced by diabetes mellitus.

Dysfunctional endothelial cells are responsible for a decrease in nitric oxide release which leads to a reduction of arterial inflow into corporal tissues. The invention demonstrates that endothelial progenitor cells (EPC) obtained from peripheral blood can be used to obtain therapeutic endothelial cells when injected into diseased corpora, such cells can restore normal erectile function in a diabetic model. This cell-based technology can be used as a treatment modality for diabetic patients with erectile dysfunction.

In one aspect, the invention discloses methods for ameliorating erectile dysfunction (ED) in a subject in need thereof, comprising isolating endothelial cells, injecting the endothelial cells into corporal tissue and allowing the cells to integrate into the tissue and increase local tissue concentration of nitric oxide, and maintaining the cells in vivo to improve intracavernous pressure upon stimulation. The EPC can be isolated from peripheral blood or bone marrow. The cells can be allogenic. In some preferred embodiments, the cells are autologous. The endothelial progenitor cells can be differentiated ex vivo to produce endothelial cells. The endothelial cells can be isolated and expanded in culture. The expanded cells can be injected directly into the corpus cavernosum. Approximately 0.1 to about 10 million cells can be implanted per injection. The concentration of cells used can vary depending on the extent of ED present in the subject. Injections can be repeated if necessary.

The method can be used in a subject that has diabetes mellitus or type 2 diabetes. In order to maintain maximum benefit from the cell therapy, the subject can also be counseled to control blood levels of glucose. Ideally, the blood glucose levels are maintained within the normal range for the subject.

In some embodiments, smooth muscle cells may be used to restore smooth muscle function. In other embodiments, EPC and progenitor muscle cells (MPC) can be combined and injected into the corporal tissue to enhance corporal tissue function. The EPC and MPC can both be isolated from peripheral blood. The cells can be obtained from somatic or stem cells.

In another aspect of the invention, a desired cell population for injection into a subject can be isolated from a body fluid, such as peripheral blood, using at least one physical property of the desired population of cells or at least one cell specific affinity moiety. Physical properties can include size filtration or active cell sorting by fluorescent or magnetic labeling. Affinity moieties useful to attract a desired cell population, can include, for example, antibodies, protein ligands, nucleic acids or peptides. In a preferred embodiment, the cell specific affinity moiety includes cell specific antibodies. In some embodiments, progenitor cells can be isolated using the methods of the invention. For example, the affinity moiety can be selected to have substantial affinity for a specific progenitor cell surface marker, such as CD133, CD31, CD34, sca-1, von Willebrand factor (vWF) or c-kit. This enables purification of large quantities of peripheral progenitor cells for expansion and/or differentiation before administration to the subject. Further details on cell isolation techniques can be found in commonly-owned, co-pending, U.S. Provisional Patent Application No. 61/022,028 filed January 18, 2008, entitled "Methods Of Isolating And Purifying Cells For Therapy".

In yet another aspect of the invention the population of cells to be administered to the corporal tissue can be encapsulated. In one embodiment, encapsulation can be in the form of microspheres, e.g., alginate-Poly-L-lysine capsules which allow nutrients to reach the immunoprotected cells, while the angiogenesis modulating agent proteins secreted from the cells diffused into the surrounding tissues. The microspheres protect the coated cells from the host immune environment. Moreover, as noted above, the cells can also be engineered to produce factors that further enhance nitric oxide production and/or modulate blood glucose levels. Further details on cell isolation techniques can be found in commonly-owned, co-pending, U.S. Patent Application Ser. No. 10/766,642 filed January 28, 2004, (Pub. No. US2005-0002915) entitled "Enhancement Of Angiogenesis To Grafts Using Cells Engineered To Produce Growthfactors".

### Description of the Drawings

Figure 1 is a schematic showing the physiology of normal erectile function;
Figure 2 is a schematic showing the overall study design described in Example demonstrating that progenitor cell derived endothelial cell therapy can be used to treat erectile dysfunction;
Figure 3 is a graph of blood glucose over time showing the comparison between the diabetes group and cellular therapy group;
Figure 4A is a graph of the results from cavernosometry showing the intracavernous pressure in normal rats;
Figure 4B is a graph of the results from cavernosometry showing the intracavernous pressure in diabetic rats;
Figure 4C is a graph of the results from cavernosometry showing the intracavernous pressure in diabetic rats 12 weeks following cell therapy with endothelial progenitor cells;
Figure 5 is a graph of the ratio of ICP (intracavernous pressure)/MAP (mean arterial pressure) over time comparing the intracavernous pressure results of the diabetes group (DB), diabetes group with cell therapy with endothelial cells (CT), diabetes group with insulin treatment (Insulin) and diabetes group with cell therapy with endothelial progenitor cells and insulin treatment (CT + Insulin), following 4 weeks of diabetes induction;
Figure 6 is a graph of the ratio of MAP (mean arterial pressure)/ ICP (intracavernous pressure) versus neurostimulation comparing the cavernosometry results of the diabetes and normal group 8 weeks after diabetes induction;
Figure 7 is a graph of the ratio of MAP (mean arterial pressure)/ ICP (intracavernous pressure) over time comparing the cavernosometry results of the diabetes group (DB) and diabetes group with cell therapy with endothelial progenitor cells (DB+CT), following 8 weeks of diabetes induction; and
Figure 8 is a graph of the ratio of ICP (intracavernous pressure)/ MAP (mean arterial pressure) comparing the results for normal rats, diabetic rats (DM), and diabetic rats 12 weeks following cell therapy with endothelial progenitor cells (DM+CT).

### Detailed Description

So that the invention may more readily be understood, certain terms are first defined:
The term "isolating" or "isolated" as used herein refers to the process of fractionating cells or cells that have been fractionated into subpopulations from which the cells elements can be obtained. This also may be accomplished using standard techniques for cell separation used by persons skilled in the art of cell culture.
The term "differentiation" or "differentiated" as used herein refers to maturation of a progenitor cell or a cell that is more mature than the progenitor cell from which it was derived.
The term "subject" as used herein is intended to include living organisms in which an immune response is elicited. Preferred subjects are mammals. Examples of subjects include but are not limited to, humans, monkeys, dogs, cats, mice, rates, cows, horses, pigs, goats and sheep.

### I. Erectile Dysfunction (ED)

ED is the persistent inability to attain or maintain an erection sufficient for sexual performance. In a relaxed state, a balance exists between blood flow in and out of the erectile bodies. Normal erectile function requires a complex set of dynamic neural and vascular interactions. Penile erection can be elicited by multiple mechanisms (e.g., central psychogenic and reflexogenic). These mechanisms interact during normal sexual activity. Psychogenic stimili include auditory, visual, olfactory or imaginary stimuli, and reflexogenic stimuli involve sensory receptors on the penis that cause somatic and parasympathetic efferent actions.

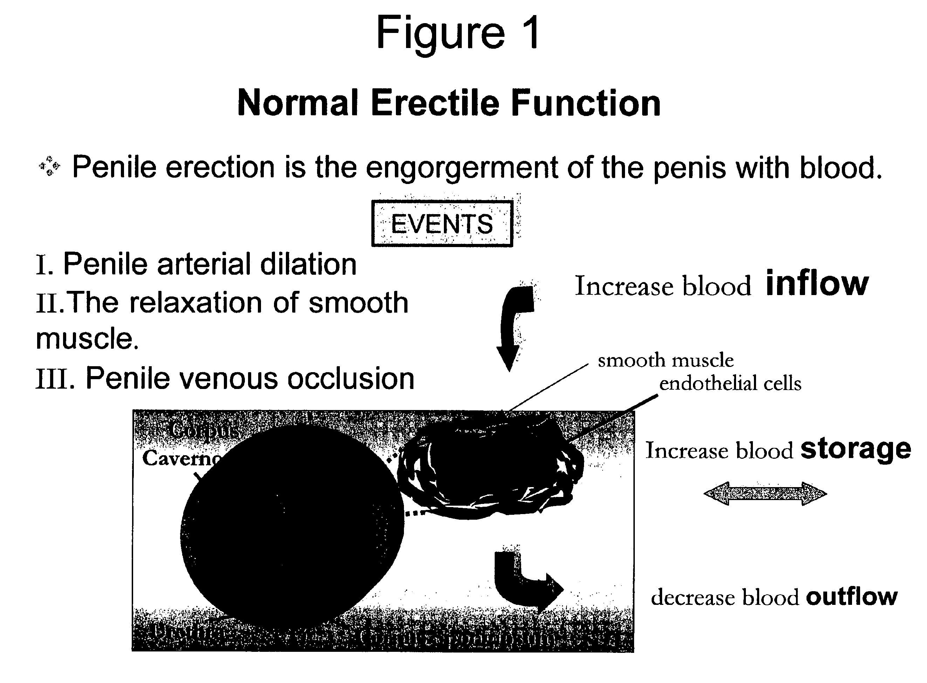

Upon arousal, parasympathetic activity triggers a series of events that start with the release of nitric oxide and end with increased levels of the intracellular mediator cyclic guanosine monophosphate (cGMP). Increases in cGMP cause penile vascular and trabecular smooth muscle relaxation, which leads to increased blood flow into the corpora cavernosa. The rapid filling of the cavernosal spaces compresses venules resulting in decreased venous outflow (e.g., the corporeal veno-occlusive mechanism). The combination of increased inflow and decreased outflow rapidly raises intracavernosal pressure resulting in progressive penile rigidity and full erection. A schematic of normal erectile function is shown in Figure 1.

While ED can have either psychogenic or organic causes, the majority of cases have at least some organic cause, such as vasculogenic, neurogenic and hormonal causes. Approximately 35-75 % of men with diabetes mellitus are afflicted with ED, which accounts for approximately 30% of ED cases in the United States. ED in men with diabetes has a multifactorial etiology and have been associated with endothelial cell dysfunction, neuropathy, vascular disease, endocrine disorders, diabetes control and medication. The specific mechanisms and associated impairments include, for example, hyperglycemia which can lead to glycation of elastic fibers and failure of relaxation of the corpora cavernosa, endothelial dysfunction of the sinosoidal endothelial cells that can result in a decreases in nitric oxide release and impaired vasodilatation, and peripheral vascular disease that can result in reduced arterial and arteriolar inflow.

In one aspect of the invention, methods of ameliorating ED using cell therapy is disclosed. Endothelial progenitor cells (EPC) injected into the corporal tissue are shown to have a long term effect towards improving ED. The methods of the invention can be used to increase local nitric oxide concentrations. The EPC can be obtained from peripheral blood, preferably from the patient to be treated. In another embodiments, mesenchymal stem cells can be used. In yet other embodiments, a combination of different cell types can be injected into the corporal tissue. For example, EPC and smooth muscle cells can be used as described below. In yet other embodiments, the cells can be combined with growth factors, such as vascular endothelial growth factor (VEGF), fibroblast growth factor (FGF), transforming growth factor-alpha and -beta (TGF), and platelet derived growth factor (PDGF).

The methods of the invention provide long term therapy for males with ED. For example, improvements have been seen up to 12 weeks following cell therapy. In patients with diabetes, the cell therapy methods of the invention have been shown to have longer effects if blood glucose is controlled (e.g., through the of use of medication such as insulin, diet, and exercise).

The amount of cells injected will vary based on the degree of disease, and condition, and weight of the subject to be treated. For example, about 0.3 to about 10 million cells per injection can be injected into the corpus cavernosum. Injections can be repeated, if necessary.

### II. Culturing Cells

The endothelial cells can be isolated from allogenic cell populations derived from the same species or autogenic derived from the subject□s own tissue. The cells can also be xenogenic, where cell populations are derived from a mammalian species that are different from the subject. For example, tissue cells can be derived from mammals such as monkeys, dogs, cats, mice, rats, cows, horses, pigs, goats and sheep.

The isolated cells are preferably cells obtained from a peripheral blood sample or biopsy, from the subject's own tissue. A biopsy can be obtained by using a biopsy needle under a local anesthetic, which makes the procedure quick and simple. The small biopsy can then be differentiated and expanded in culture to obtain the tissue cells. Cells from relatives or other donors of the same species can also be used with appropriate immunosuppression.

Methods for the isolation and culture of cells are discussed by Freshney, Culture of Animal Cells. A Manual of Basic Technique, 2d Ed., A. R. Liss, Inc., New York, 1987, Ch. 9, pp. 107-126. Cells may be isolated using techniques known to those skilled in the art. For example, the tissue can be cut into pieces, disaggregated mechanically and/or treated with digestive enzymes and/or chelating agents that weaken the connections between neighboring cells making it possible to disperse the tissue into a suspension of individual cells without appreciable cell breakage. If necessary, enzymatic dissociation can be accomplished by mincing the tissue and treating the minced tissue with any of a number of digestive enzymes either alone or in combination. These include but are not limited to trypsin, chymotrypsin, collagenase, elastase, and/or hyaluronidase, DNase, pronase, and dispase. Mechanical disruption can also be accomplished by a number of methods including, but not limited to, scraping the surface of the tissue, the use of grinders, blenders, sieves, homogenizers, pressure cells, or insonators to name but a few.

Cell types include, but are not limited to, endothelial cells such as human endothelial cells, progenitor cells isolated from the peripheral blood or bone marrow that can be induced to differentiate into different cells, stem cells, committed stem cells, and/or differentiated cells may be used. In a preferred embodiment, endothelial progenitor cells are autologously derived from peripheral blood.

Also, depending on the type of tissue or organ being made, specific types of committed stem cells can be used. For instance, myoblast cells can be used to build various muscle structures. Other types of committed stem cells can be used to make organs or organ-like tissue such as heart, kidney, liver, pancreas, spleen, bladder, ureter and urethra. Other cells include, but are not limited to, endothelial cells, muscle cells, smooth muscle cells, fibroblasts, osteoblasts, myoblasts, neuroblasts, fibroblasts, glioblasts; germ cells, hepatocytes, chondrocytes, keratinocytes, cardiac muscle cells, connective tissue cells, epithelial cells, endothelial cells, hormone-secreting cells, cells of the immune system, neurons, cells from the heart, kidney, liver, pancreas, spleen, bladder, ureter and urethra, and the like. In some embodiments it is unnecessary to pre-select the type of stem cell that is to be used, because many types of stem cells can be induced to differentiate in an organ specific pattern once delivered to a given organ. For example, a stem cell delivered to the liver can be induced to become a liver cell simply by placing the stem cell within the biochemical environment of the liver.

Examples also include cells that have been genetically engineered, transformed cells, and immortalized cells. One example of genetically engineered cells useful in the present invention is a genetically engineered cell that makes and secretes one or more desired molecules. When genetically engineered cells are implanted in an organism, the molecules produced can produce a local effect or a systemic effect, and can include the molecules identified above as possible substances.

Cells may produce substances that inhibit or stimulate inflammation; facilitate healing; resist immunorejection; provide hormone replacement; replace neurotransmitters; inhibit or destroy cancer cells; promote cell growth; inhibit or stimulate formation of blood vessels; augment tissue; and to supplement or replace the following tissue, neurons, skin, synovial fluid, tendons, cartilage, ligaments, bone, muscle, organs, dura, blood vessels, bone marrow, and extracellular matrix. Other factors and differentiation inducers may be added to the culture to promote specific types of cell growth.

Once the tissue has been reduced to a suspension of individual cells, the suspension can be fractionated into subpopulations from which the cells elements can be obtained. This also may be accomplished using standard techniques for cell separation including, but not limited to, cloning and selection of specific cell types, selective destruction of unwanted cells (negative selection), separation based upon differential cell agglutinability in the mixed population, freeze-thaw procedures, differential adherence properties of the cells in the mixed population, filtration, conventional and zonal centrifugation, centrifugal elutriation (counterstreaming centrifugation), unit gravity separation, countercurrent distribution, electrophoresis and fluorescence-activated cell sorting (*see e.g.*, Freshney, (1987) Culture of Animal Cells. A Manual of Basic Techniques, 2d Ed., A. R. Liss, Inc., New York, Ch. 11 and 12, pp. 137-168). For example, salivary cells may be enriched by fluorescence-activated cell sorting. Magnetic sorting may also be used.

Cell fractionation may also be desirable, for example, when the donor has diseases such as cancer or tumor. A cell population may be sorted to separate the cancer or tumor cells from normal noncancerous cells. The normal noncancerous cells, isolated from one or more sorting techniques, may then be used for tissue reconstruction.

Growth factors and regulatory factors can be added to the media to enhance, alter or modulate proliferation and cell maturation and differentiation in the isolated cell cultures. The growth and activity of cells in culture can be affected by a variety of growth factors such as growth hormone, somatomedins, colony stimulating factors, erythropoietin, epidermal growth factor, hepatic erythropoietic factor (hepatopoietin), and like. Other factors which regulate proliferation and/or differentiation include prostaglandins, interleukins, and naturally-occurring chalones.

Differentiated cells can be cultured *in vitro* to increase the number of cells available for injection. Differentiated cells that have been expanded can also undergo an additional isolation step to obtain one or more populations of cells. To prevent an immunological response after implantation of the cells, the subject may be treated with immunosuppressive agents such as, cyclosporin or FK506.

Differentiated cells may be transfected with a nucleic acid sequence. Useful nucleic acid sequences may be, for example, genetic sequences which reduce or eliminate an immune response in the host. For example, the expression of cell surface antigens such as class I and class II histocompatibility antigens may be suppressed. In addition, transfection could also be used for gene delivery. Cells may be transfected with specific genes prior to seeding onto the biocompatible substitute. Thus, the cultured cells can be engineered to express gene products that would produce a desired protein that helps ameliorate a particular disorder.

The cells grown may be genetically engineered to produce gene products beneficial to implantation, e.g., anti-inflammatory factors, e.g., anti-GM-CSF, anti-TNF, anti-IL-1, and anti-IL-2. Alternatively, the endothelial cells may be genetically engineered to "knock out" expression of native gene products that promote inflammation, *e.g.*, GM-CSF, TNF, IL-1, IL-2, or "knock out" expression of MHC in order to lower the risk of rejection.

Methods for genetically engineering cells for example with retroviral vectors, adenoviral vectors, adeno-associated viral vectors, polyethylene glycol, or other methods known to those skilled in the art can be used. These include using expression vectors which transport and express nucleic acid molecules in the cells. (*See* Geoddel; Gene Expression Technology: Methods in Enzymology 185, Academic Press, San Diego, CA (1990).

Vector DNA is introduced into prokaryotic or eukaryotic cells via conventional transformation or transfection techniques. Suitable methods for transforming or transfecting host cells can be found in Sambrook et al. Molecular Cloning: A Laboratory Manual, 2nd Edition, Cold Spring Harbor Laboratory press (1989), and other laboratory textbooks.

The cells of the invention can be used in a variety of applications. For example, the endothelial cells can be implanted into a subject to replace or augment existing tissue. The subject can be monitored after implantation of the endothelial cells, for amelioration of the disorder.

The cells can be used *in vitro* to screen a wide variety of compounds, for effectiveness and cytotoxicity of pharmaceutical agents, chemical agents, growth/regulatory factors. The cultures can be maintained *in vitro* and exposed to the compound to be tested. The activity of a cytotoxic compound can be measured by its ability to damage or kill cells in culture. This may readily be assessed by vital staining techniques. The effect of growth/regulatory factors may be assessed by analyzing the cellular content after the injection, e.g., by total cell counts, and differential cell counts. This may be accomplished using standard cytological and/or histological techniques including the use of immunocytochemical techniques employing antibodies that define type-specific cellular antigens. The effect of various drugs on normal cells cultured in the artificial tissue may be assessed.

### Examples

### Example 1: Materials

Unless otherwise stated, all chemicals were purchased from Sigma (St. Louis, MO). ECL buffers and 125I-Sodium were purchased from PerkinElmer NEN (Boston, MA). Collagenase type II (1 mg/ml) was purchased from Boehringer Mannheim (Mannheim, Germany). Endothelial medium (EBM-2) was purchased from Cambrex Bio Science (Walkersville, MD). PCR reagents and primers, M199 medium, fetal bovine serum (FBS) and penicillin were purchased from Life Technologies (Gaithersburg, MD). Basic Fibroblast Growth Factor (bFGF) was a gift from Judith Abraham, (Scios Nova, CA). FITC-labeled monoclonal anti human CD31 antibodies were purchased from Santa Cruz (Santa Cruz, CA). Rabbit polyclonal anti von Willebrand factor (vWF), anti smooth muscle actin antibodies, and FITC labeled anti-rabbit IgG antibodies were purchased from DAKO (Glostrup, Denmark). Monoclonal anti human CD31, Goat polyclonal anti-VE Cadherin (VE-Cad), Goat anti-vWF and Rabbit polyclonal anti Flk-1 antibodies were purchased from Santa Cruz (Santa Cruz, CA). FITC and Biotin-labeled Ulex europeaus I lectin was purchased from Sigma (St. Louis, MO). Biotin-labeled goat anti-mouse IgG and biotin-labeled mouse anti-goat IgG were purchased from Santa Cruz (Santa Cruz, CA). Anti-CD105 antibodies were purchased from BD Pharmingen (San Diego, CA). FITC-conjugated avidin was purchased from Vector Laboratories (Burlingame, CA).

### Example 2: Injection of Endothelial Progenitor Cells For Ameliorating Erectile Dysfunction

This study demonstrates that EPC obtained from peripheral blood can be used to achieve mature endothelial cells. The endothelial cells injected into diseased corpora are able to restore normal erectile function in a diabetic rat model. This cell-based technology may be a viable treatment modality for diabetic patients with erectile dysfunction.

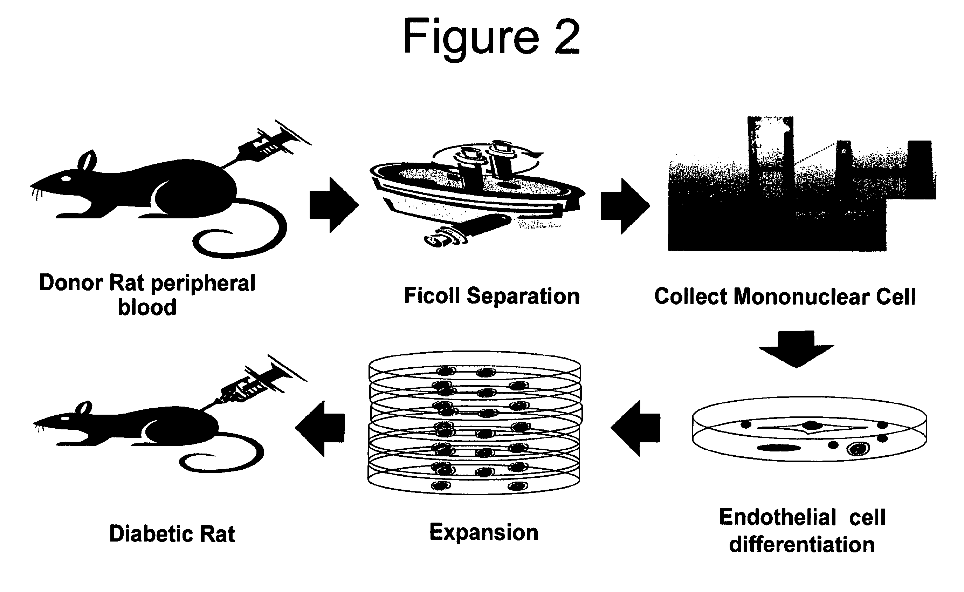

A model of Diabetes was created by intraperitoneal injection of Streptozotocin (50 mg/Kg). Those animals with a blood glucose level of greater than 300 mg/dl were considered as diabetics and used for this study. EPC were isolated from peripheral blood of donor rats. The cells were grown, expanded and induced into an endothelial cell lineage. The cells were characterized using cell-specific markers (CD31, CD34, vWF, CD133). The culture expanded endothelial cells were labeled with red fluorescent dye (PKH26) and injected directly into the dysfunctional corpora, and the animals were followed for up to 12 weeks. Erectile function was assessed by intracavernous pressure and the corporal tissues were retrieved for histo- and immunohistochemical analyses. Figure 2 shows a schematic of the overall study design.

### (i) Diabetes Induction, treatment and anesthesia.

84 male Lewis rats weighing around 200-250 g, were breed and grown in at Wake Forest University. The animals had free access to food and water and special care for diabetics animals. All experimental procedures were reviewed and approved by the Wake Forest University Institutional Animal Care and Use Committee (IACUC) and were performed in compliance with the Animal Welfare Act and the guide for the Care and Use of Laboratory Animals. The animals were housed at the animal facilities of the Wake Forest University with a 12 hour light cycle.

Diabetes was induced by 50 mg/Kg intraperitoneal streptozotocin (STZ) dissolved in sterile buffer solution of sodium phosphate 0.2 M and citric acid 0.1 M.

Before the injection, tail blood glucose was measured to obtain the basal values. After the injection, tail blood glucose was measured for three consecutive days in order to confirm the hyperglycemia. If the rats did not become diabetic after the first STZ injection, those rats received second STZ injection in the following week. Animals with a blood glucose level of 300 mg/dL or more were considered as diabetics and used for the subsequent experiments. Blood glucose levels and body weight were monitored each week for 8-11 weeks using a glucose test kit and these diabetics rats were divided in four groups:
GROUP I: Diabetics Lewis rats (n=20).
GROUP II: Diabetics Lewis rats with Cell Therapy (n=20).
GROUP III: Diabetics Lewis rats with insulin treatment (n=20).
GROUP IV: Diabetics Lewis rats with insulin treatment and Cell Therapy (n=20).

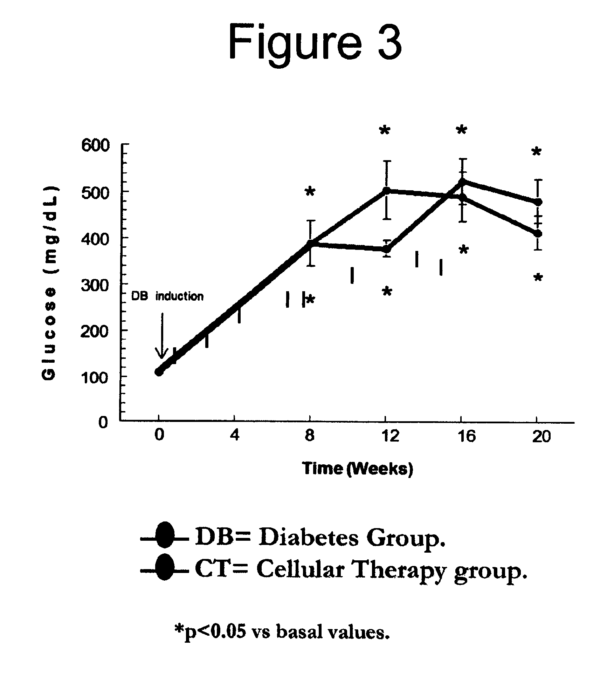

Additionally, 10 age-matched Lewis rats were used as normal control. All animals received the treatment established for each group during another 12 weeks. Figure 3 is the diabetic standardization curve showing a graph of blood glucose over time for the diabetes group and diabetes group with cellular.

### (ii) Isolation of EPC.

Mononuclear cells were isolated from 10 mL of peripheral blood of syngenic donor Lewis rats by density-gradient centrifugation with histopaque 1077 (Sigma). Immediately after isolation, the mononuclear cells were plated on 6-well culture dishes coated with human fibronectin (Sigma) at 5x10⁶ cells concentration in each one, maintained in Endothelial Basal Medium (EBM, CellSystems) supplemented with EGM SingleQuots, containing 2% Fetal Bovine Serum, Human VEGF-1, Human fibroblast growth factor-2 (FGF-2), Human epidermal growth factor (EGF), Insulin-like growth factor-1 (IGF-1), and Ascorbic acid and incubate for two days at 37°C, 5% CO₂ with ≥ 95% humidity.

After two days, the non-adherent cell were harvested and cultured at 1x10⁶ cells concentration on fibronectin-coated 24-well dishes and incubated for an additional 3 days to allow formation of endothelial colonies.

The vast majority of these ex vivo expanded cells were of endothelial lineage and, as such, they constituted the ex vivo expanded EPC-enrich fraction.

The cells were isolated, grown, differentiated and expanded until a sufficient number of cells is obtained for implantation (8 weeks). The implanted cells were in passage 7, 8 and 9.

### (iii) Examination in Vitro.

The cellular characterization was done with the examination of the expression of cell-surface markers CD31, CD34, vWF and CD133 at a dilution of 1:20. Immunolabeling was done using the avidin-biotin detection kit (Vectastain elite ABC, Vector Laboratories Inc, Bulingame, CA) and by immunofluorescence at a dilution of 1:200 for the second antibody.

### (iv) Labeling with PKH26

EC's were labeled with red fluorescence PKH26-red using a cell linker kit (PKH26, Sigma, Saint Louis, MI) according to the manufacturers guidelines. Briefly, cells in PBS were added to a 10 uM dye solution and gently mixed. After an incubation period of 5 min at 25°C FBS was added to stop the staining reaction. The staining solution was removed by repetitive washes with PBS. After conformation of flourescence by microscopy the cells were placed on ice until injection

### (v) Transplantation of EPC

The rats were anesthetized using an anesthesia station (VetEquip, Inc. Pleasanton, CA) with 1-2% of inhaled Isoflurane (Abbott) mixed with 3 L/min O₂. The rats were placed in a supine position on a thermoregulated surgical table. The ventral abdominal wall and perineum were shaved with an electric shaver and cleaned with betadine. By using a sterile technique, a midline incision in the perineum was made. The corpus cavernosum was dissected by blunt dissection and exposed. By using a 30-gauge needle attached to a microliter syringe, 1-2x10⁶ cells diluted in 20 µl of EGM-2 were injected into the corpus cavernosum. The rats from the diabetic control group (Group I) received only 0.2 mL of EGM-2 as vehicle.

### (vi) Treatment with insulin.

The diabetic rats from groups III and IV received treatment with injection of 5-10 UI of insulin (Eli Lilly, Indianapolis, IN, U.S.A.), subcutaneous every day, with the propose to maintain glucose blood levels into normal range.

### (vii) Results

### Intracavernous pressure in normal and diabetics rats.

For the surgical technique to assess the intracavernosal pressure, at 4, 8 and 12 weeks after of treatment, 5 rats of each group were anaesthetized with an i.p. injection (35 mg/kg) of sodium pentobarbital. Anaesthesia was maintained during the course of the experimental protocol (2-3 h), as necessary, by subsequent injection of pentobarbital (5-10 mg/kg) every 45-60 min as required. The neck, ventral abdominal wall and perineum were shaved with an electric shaver and cleaned with betadine.

The carotid artery was exposed through an anterior-midline neck incision and cannulated with a polyethylene catheter 19 fr filled with physiological saline solution and connected to a pressure transductor to continuously monitor the mean arterial pressure (MAP).

The pressure transducer was calibrated in cmH₂O before each experiment. A lower midline incision was made through the lower abdomen, and the bladder and prostate identified. The inferior hypogastric plexus (i.e. the pelvic plexus or major pelvic ganglia), pelvic nerves and the cavernosal nerve were identified posterolateral to the prostate on both sides, and stainless-steel bipolar wire electrodes were placed around these structures for electrical stimulation. The determination of rat "erection" is through the measurement of cavernosal pressure. In humans and animals the pressure is initially very low (approx. 5-10 mmHg), and during erection it rises to 60-90% of systemic blood pressure (depending on the species and needle placement).

The penis was then denuded of skin; both crura (corpus cavernosum) were exposed by removing part of the overlying ischiocavernosal muscle. To monitor the ICP, a 20-G cannula was filled with 250 U/mL of heparin solution, connected to polyethylene-50 tubing (Intramedic, Becton Dickinson, CA, USA) and inserted into the right corpus cavernosum. The pressure transduce were calibrated in cmH₂O before each experiment.

Both pressure lines were connected to a pressure transducer, and then via a transducer amplifier (ETH 401) to a data acquisition board, with real-time display and recording of ICP (PowerLab. Chart 5, ADInstruments).

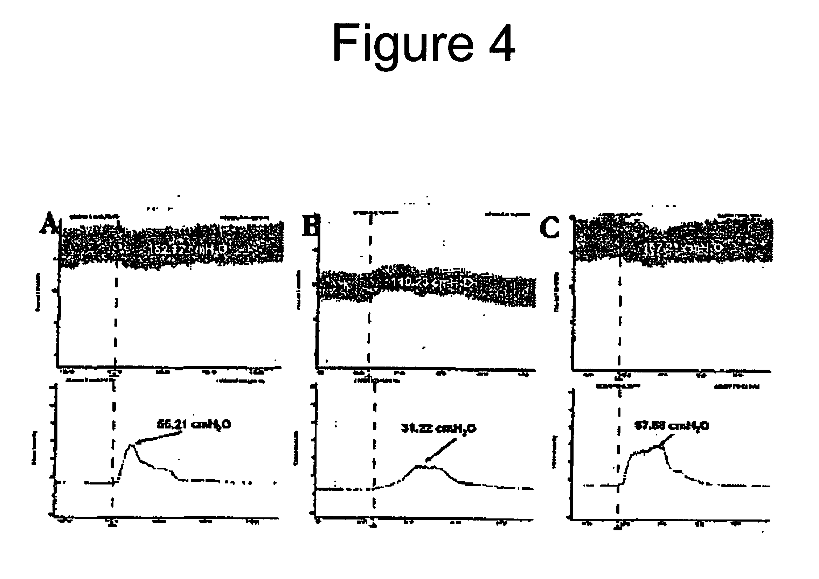

The cavernosal nerve was directly electrostimulated as previously described with a delicate stainless-steel bipolar hook electrode attached to the multi-jointed clamp. Each probe was 0.2 mm in diameter; the two poles were separated by 1 mm. Monophasic rectangular pulses were delivered by a signal generator (custom-made and with integral constant-current amplifier) (Grass, Astro Med, Inc. W. Warwick, RI, U.S.A.). The stimulation parameters were: 20 Hz, pulse width 0.22 ms, and duration 1 min; increasing current of 1, 2, 4 and 6 mA was used. The stimulus interval was 10 min. These parameters were recorded on a polygraph and the data acquisition and calculation of the derived parameters were performed using a software computer system (See Figures 4A-C top: mean arterial pressure (MAP), bottom: intracavernosal pressure (ICP)).

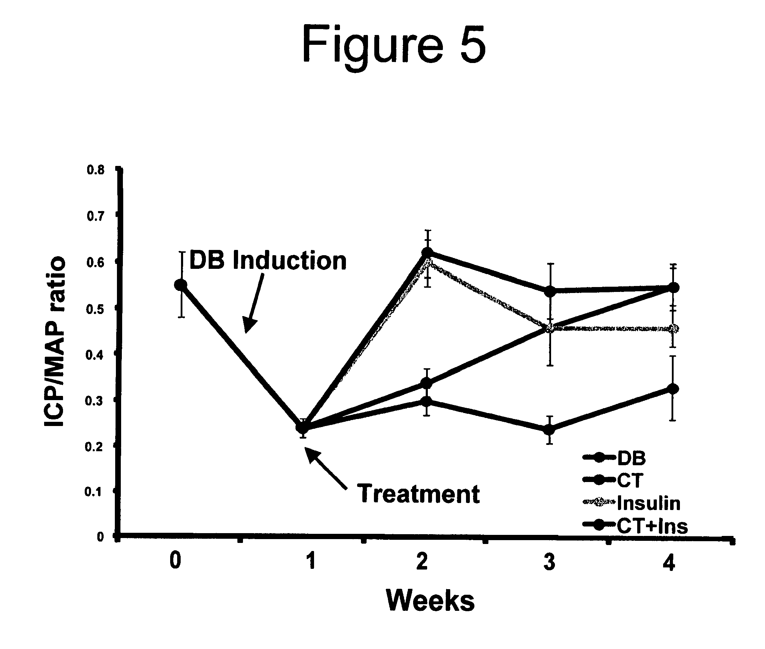

Figure 5 is a graph of the ratio of ICP (intracavernous pressure)/MAP (mean arterial pressure) versus time comparing the intracavernous pressure over time of the all four groups diabetes group (DB), diabetes group with cell therapy with endothelial cells (CT), diabetes group with insulin treatment (Insulin) and diabetes group with cell therapy with endothelial progenitor cells and insulin treatment (CT + Insulin) 4 weeks after diabetes induction.

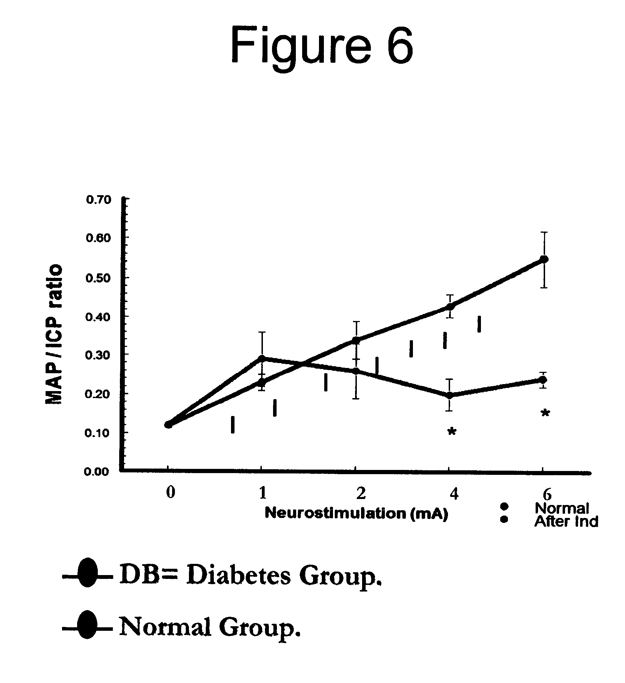

Figure 6 is a graph of the ratio of MAP (mean arterial pressure)/ ICP (intracavernous pressure) versus neurostimulation comparing the cavernosometry results of the diabetes and normal group 8 weeks after diabetes induction.

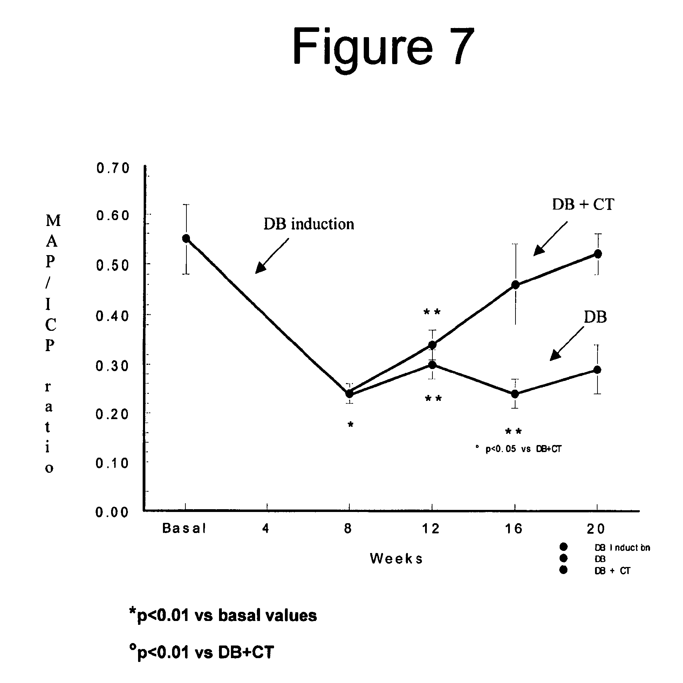

Figure 7 shows a graph of MAP / ICP over time comparing the cavernosometry results of the diabetes group (DB) and diabetes group with cell therapy with endothelial progenitor cells (DB+CT), following 8 weeks of diabetes induction. The MAP/ICP in the cell therapy group had continuous improvement up 12 weeks following injection indicating the long term effect of the therapy.

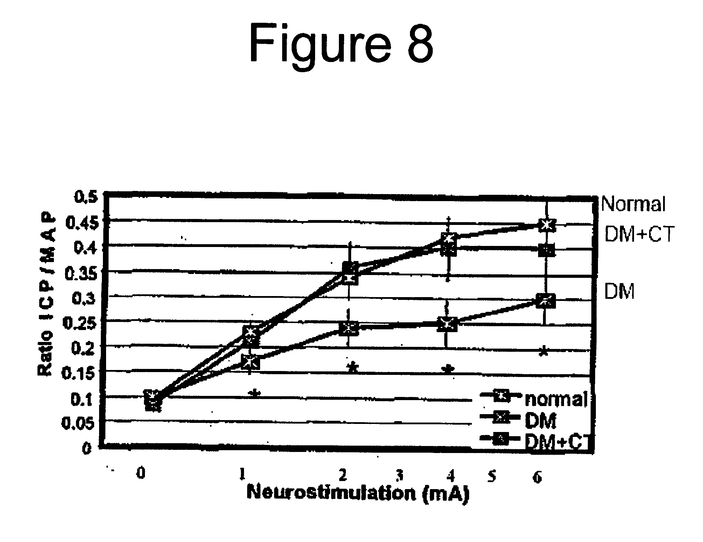

At the end of the study (12 weeks following EPC injection), the ratio ICP/MAP with different amounts of neurostimulation was compared for the normal, diabetes (DM) and diabetes with cell therapy (DM+CT) groups as shown in Figure 8 (results are presented in mean values ± sem. *p<0.05 vs normal group). After 12 weeks following cell therapy the DM+CT closely resembled the results of the normal group. The results indicate the long term effects of the endothelial progenitor cell therapy.

### Histology and Immunofluorescence.

After the cavernosomerty, the penis was excised at its base and the glans penis and connective tissue surrounding the shaft were removed for histological studies. The retrieved tissue samples were placed in Tissue-Tek® O.C.T. Compound 4583 (Sakura®) and frozen in liquid nitrogen. The frozen blocks were sectioned into 6 µm slices using a cryostat (Model CM 1850, Leica Microsystems, Bannockburn, IL). The slides were fixed and stained with hematoxylin and eosin (H&E).

PKH26 labeled cells were identified by fluorescence microscopy using an excitation wavelength of 550 nm. Digital images were taken (Zeiss Axio Imager M1 Microscope, Carl Zeiss, Thornwood, NY) at varying magnifications. The implanted cells labeled with PKH26 could be detected in the corpora tissue after 4, 8, and 12 weeks indicating long term survival of the cells.

EPC-derived endothelial cells were successfully isolated, grown and expanded.

The progenitor and differentiated endothelial cell phenotypes were confirmed using immunohistochemistry with cell specific antibodies. The animals injected with cells showed a substantial improvement in intracavernous pressure and their erectile function was restored to normal levels. Histologically, the implanted cells that were labeled with the fluorescent dye tracer PKH26 survived and integrated into the corporal tissue within the injected region.

## Claims

1. A method of preparing a medicament for the treatment of erectile dysfunction, comprising:
isolating endothelial progenitor cells from a sample;
differentiating the progenitor cells ex vivo to produce endothelial cells;
expanding the endothelial cells; and
preparing 0.1 to 10 million of the expanded endothelial cells as an injectable composition suitable for implantation into corporal tissue of a subject.

2. The method of claim 1, wherein the sample is an autologous sample, preferably from peripheral blood or bone marrow.

3. The method of claim 1, wherein the step of isolating progenitor cells further comprises separating the progenitor cells based on their exhibition of at least one marker selected from the group of CD133, CD31, CD34, sca-1, von Willebrand factor and c-kit.

4. The method of claim 3, wherein the step of isolating progenitor cells further comprises separating the progenitor cells with an affinity moiety to one or more of the markers and, preferably, the affinity moiety is selected from the group consisting of antibodies, protein ligands, nucleic acids and peptides.

5. The method of claim 1 further comprises:
isolating muscle progenitor cells from the sample;
differentiating the muscle progenitor cells ex vivo to produce muscle cells;
expanding the muscle cells; and
preparing the expanded muscle cells and the expanded endothelial cells as an injectable composition suitable for implantation into corporal tissue of the subject.

6. The method of claim 1, wherein the method further comprises isolating smooth muscle cells from the sample and the injectable population further comprises the isolated smooth muscle cells.

7. The method of claim 1, wherein the step of preparing the expanded endothelial cells further comprises encapsulating the expanded endothelial cells prior to formulation as an injectable composition.

8. The method of claim 7, wherein the expanded endothelial cells are encapsulated in alginate-Poly-L-lysine microspheres.

9. A composition for use in the treatment of erectile dysfunction, comprising:
0.1 to 10 million endothelial cells capable of implantation, derived from isolated endothelial progenitor cells differentiated ex vivo to produce endothelial cells; further **characterized in that** the endothelial cells are expanded ex vivo and formulated as an injectable composition suitable for implantation into corporal tissue of a subject.

10. The composition for use according to claim 9, wherein the endothelial cells are derived from isolated peripheral blood or bone marrow endothelial progenitor cells.

11. The composition for use according to claim 9, wherein the isolated endothelial progenitor cells are isolated from a sample previously obtained from the subject.

12. The composition for use according to claim 9, wherein the injectable composition further comprises smooth muscle cells derived from isolated muscle progenitor cells.

13. The composition for use according to claim 9, wherein the endothelial cells are encapsulated.

14. The composition for use according to claim 13, wherein the endothelial cells are encapsulated in alginate-Poly-L-lysine microspheres.

## Patentansprüche

1. Verfahren zur Herstellung eines Medikaments zur Behandlung der erektilen Dysfunktion, umfassend:
Isolieren der endothelialen Vorläuferzellen aus einer Probe;
ex vivo Differenzieren der Vorläuferzellen, um endotheliale Zellen herzustellen;
Expandieren der endothelialen Zellen; und
Zubereiten von 0,1 bis 10 Millionen der expandierten endothelialen Zellen als eine injizierbare Zusammensetzung, die geeignet zur Einpflanzung in Körpergewebe eines Subjekts ist.

2. Verfahren gemäß Anspruch 1, wobei die Probe eine autologe Probe, bevorzugt aus peripherem Blut oder Knochenmark, ist.

3. Verfahren gemäß Anspruch 1, wobei der Schritt des Isolierens der Vorläuferzellen weiter das Separieren der Vorläuferzellen, 1 basierend auf ihrer Vorzeigung von mindestens einem Marker, ausgewählt aus der Gruppe von CD133, CD31, CD34, sca-1, von Willebrand-Faktor und c-Kit, umfasst.

4. Verfahren gemäß Anspruch 3, wobei der Schritt des Isolierens der Vorläuferzellen weiter das Separieren der Vorläuferzellen mit einer Affinitätseinheit gegen einen oder gegen mehrere der Marker umfasst und, bevorzugt, die Affinitätseinheit ausgewählt ist aus der Gruppe, bestehend aus Antikörpern, Protein-Liganden, Nucleinsäuren und Peptiden.

5. Verfahren gemäß Anspruch 1, weiter umfassend:
Isolieren von Muskel-Vorläuferzellen aus der Probe;
ex vivo Differenzieren der Muskelvorläuferzellen, um Muskelzellen herzustellen;
Expandieren der Muskelzellen; und
Zubereiten der expandierten Muskelzellen und der expandierten Endothelzellen als eine injizierbare Zusammensetzung, die geeignet zur Einpflanzung in Körpergewebe des Subjekts ist.

6. Verfahren gemäß Anspruch 1, wobei das Verfahren weiter das Isolieren von glatten Muskelzellen aus der Probe umfasst und die injizierbare Population weiter die isolierten glatten Muskelzellen umfasst.

7. Verfahren gemäß Anspruch 1, wobei der Schritt des Zubereitens der expandierten endothelialen Zellen weiter das Verkapseln der expandierten endothelialen Zellen vor der Formulierung als injizierbare Zusammensetzung umfasst.

8. Verfahren gemäß Anspruch 7, wobei die expandierten Endothelzellen in Alginat-Poly-L-Lysin-Mikrosphären verkapselt werden.

9. Zusammensetzung zur Verwendung in der Behandlung der erektilen Dysfunktion, umfassend:
0,1 bis 10 Millionen endothelialen Zellen, fähig zur Einpflanzung, abgeleitet von isolierten endothelialen Vorläuferzellen, die ex vivo differenziert wurden, um endotheliale Zellen herzustellen;
weiter **gekennzeichnet dadurch, dass** die endothelialen Zellen ex vivo expandiert werden und als eine injizierbare Zusammensetzung, geeignet zur Einpflanzung in Körpergewebe von einem Subjekt formuliert sind.

10. Die Zusammensetzung zur Verwendung gemäß Anspruch 9, wobei die endothelialen Zellen von isolierten peripheren Blut- oder Knochenmarks-endothelialen Vorläuferzellen abgeleitet sind.

11. Die Zusammensetzung zur Verwendung gemäß Anspruch 9, wobei die isolierten endothelialen Vorläuferzellen aus einer Probe isoliert werden, die zuvor von einem Subjekt erhalten wurden.

12. Zusammensetzung zur Verwendung gemäß Anspruch 9, wobei die injizierbare Zusammensetzung weiter glatte Muskelzellen, abgeleitet von isolierten Muskel-Vorläuferzellen, umfasst.

13. Zusammensetzung zur Verwendung gemäß Anspruch 9, wobei die endothelialen Zellen verkapselt sind.

14. Zusammensetzung zur Verwendung gemäß Anspruch 13, wobei die endothelialen Zellen in Alginat-Poly-L-Lysin-Mikrosphären verkapselt sind.

## Revendications

1. Procédé de préparation d'un médicament pour le traitement d'un dysfonctionnement érectile, comprenant :
l'isolation de cellules progénitrices endothéliales d'un échantillon ;
la différenciation des cellules progénitrices ex vivo afin de produire des cellules endothéliales ;
l'expansion des cellules endothéliales ; et
la préparation de 0,1 à 10 millions de cellules endothéliales expansées sous forme de composition injectable adaptée pour une implantation dans le tissu corporel d'un sujet.

2. Procédé selon la revendication 1, dans lequel l'échantillon est un échantillon autologue, de préférence provenant de sang périphérique ou de moelle osseuse.

3. Procédé selon la revendication 1, dans lequel l'étape consistant à isoler les cellules progénitrices comprend en outre la séparation des cellules progénitrices sur la base de leur présentation d'au moins un marqueur choisi dans le groupe constitué de CD133, CD31, CD34, sca-1, facteur von Willebrand et c-kit.

4. Procédé selon la revendication 3, dans lequel l'étape consistant à isoler les cellules progénitrices comprend en outre la séparation des cellules progénitrices ayant une portion d'affinité à un ou plusieurs des marqueurs et, de préférence, la portion d'affinité est choisie dans le groupe constitué d'anticorps, de ligands de protéine, d'acides nucléiques et de peptides.

5. Procédé selon la revendication 1, comprenant en outre :
l'isolation des cellules progénitrices musculaires de l'échantillon ;
la différenciation des cellules progénitrices musculaires ex vivo afin de produire des cellules musculaires ;
l'expansion des cellules musculaires ; et
la préparation des cellules musculaires expansées et des cellules endothéliales expansées sous forme d'une composition injectable adaptée pour une implantation dans le tissu corporel d'un sujet.

6. Procédé selon la revendication 1, dans lequel le procédé comprend en outre l'isolation des cellules des muscles lisses de l'échantillon et la population injectable comprend en outre les cellules de muscles lisses isolées.

7. Procédé selon la revendication 1, dans lequel l'étape consistant à préparer les cellules endothéliales expansées comprend en outre l'encapsulation des cellules endothéliales expansées avant la formulation sous forme de composition injectable.

8. Procédé selon la revendication 7, dans lequel les cellules endothéliales expansées sont encapsulées dans des microsphères d'alginate-poly-L-lysine.

9. Composition destinée à être utilisée dans le traitement du dysfonctionnement érectile, comprenant :
0,1 à 10 millions de cellules endothéliales capables d'implantation, dérivées de cellules progénitrices endothéliales isolées différenciées ex vivo afin de produire des cellules endothéliales ; **caractérisée en outre en ce que** les cellules endothéliales sont expansées ex vivo et formulées sous forme d'une composition injectable adaptée pour une implantation dans le tissu corporel d'un sujet.

10. Composition destinée à être utilisée selon la revendication 9, dans laquelle les cellules endothéliales sont dérivées de cellules progénitrices endothéliales de sang périphériques ou de moelle osseuse.

11. Composition destinée à être utilisée selon la revendication 9, dans laquelle les cellules progénitrices endothéliales isolées sont isolées d'un échantillon précédemment prélevé sur le sujet.

12. Composition destinée à être utilisée selon la revendication 9, dans laquelle la composition injectable comprend en outre des cellules de muscles lisses dérivées des cellules progénitrices musculaires isolées.

13. Composition destinée à être utilisée selon la revendication 9, dans laquelle les cellules endothéliales sont encapsulées.

14. Composition destinée à être utilisée selon la revendication 13, dans laquelle les cellules endothéliales sont encapsulées dans des microsphères d'alginate-poly-L-lysine.
